# EUROPEAN PATENT APPLICATION

(11) **EP 3 421 480 A1**
(43) Date of publication of application: **02.01.2019**
(21) Application number: 17178896.1
(22) Date of filing: 30.06.2017
(51) Int. Cl.: C07H 1/06, C07H 5/04, C07H 13/04

(54) **METHOD FOR SEPARATING CHROMATOGRAPHICALLY INDISTINGUISHABLE AZIDONITRATES**

(71) Applicant: Sinutron KG, 1180 Vienna (AT)
(72) Inventor: The designation of the inventor has not yet been filed
(74) Representative: Sonn & Partner Patentanwälte

(57) **Abstract**

The present invention discloses a method for separating chromatographically indistinguishable 1,2-diaxial from 1,2-diequatorial disaccharide azidonitrates, wherein the disaccharide is a 1→4 disaccharide, comprising the steps of
- providing a mixture of chromatographically indistinguishable 1,2-diaxial from 1,2-diequatorial azidonitrates,
- treating the mixture with a nucleophile, selected from the group of tetraalkylammonium salts, preferably tetraalkylammonium nitrate, and
- obtaining the 1-axial-2-equatorial derivative from the treated mixture.

## Description

The present invention relates to a method for separating chromatographically indistinguishable azidonitrates.

Azidonitration (cerium ammonium nitrate and sodium azide in acetonitrile; R. U. Lemieux and R. M. Ratcliffe, Can. J. Chem., 57, 1244 - 1251) of acetylated disaccharide derivatives containing D-glucal as the reducing moiety (e.g., lactal, maltal or cellobial) affords chromatographically inseparable mixtures of D-gluco and D-manno azidonitrates which contain large amounts of (synthetically useless) side products. Some of the side products are formed from the desired D-gluco azidonitrates, resulting in diminished yields. The reaction as reported by Shing and Perlin, using cellobial **1** as an example, is shown in Scheme 1 (T. K. M. Shing and A. S Perlin, Carbohydr. Res., 130 (1984) 65 - 72).

It is an object of the present invention to overcome the chromatographical inseparability of the products of the azidonitration, preferably the inseparability of D-gluco and D-manno azidonitrates. It is a specific object of the present invention to separate chromatographically indistinguishable 1,2-diaxial from 1,2-diequatorial azidonitrates. Further objects of the present invention are the provision of purified forms of such individual chromatographically inseparable products of the azidonitration.

Therefore, the present invention provides a method for separating chromatographically indistinguishable 1,2-diaxial (i.e. α-D-manno-) from 1,2-diequatorial (i.e. β-D-gluco-) disaccharide azidonitrates, wherein the disaccharide is a 1→4 disaccharide, comprising the steps of
- providing a mixture of chromatographically indistinguishable 1,2-diaxial and 1,2-diequatorial azidonitrates,
- treating the mixture with a nucleophile, selected from the group of tetraalkylammonium salts, preferably tetraalkylammonium nitrate, (these nucleophiles do not affect the 1,2-diaxial azidonitrate but convert the 1,2-diequatorial azidonitrate into a 1-axial-2-equatorial derivative which is chromatographically distinct from the inseparable mixture and can then be separated by chromatography) and
- obtaining the 1-axial-2-equatorial derivative (i.e. the nucleophile-treated 1,2-diequatorial azidonitrate (which was converted to the 1-axial-2-equatorial derivative by the method according to the present invention)) from the treated mixture.

With the present invention, the chromatographically indistinguishable 1,2-diaxial disaccharide azidonitrates can be separated from the 1,2-diequatorial forms by selective nucleophilic reaction. The reaction products can then easily be separated, preferably by usual chromatographic steps used in common purification methods for disaccharides, e.g. flash chromatography.

The method according to the present invention surprisingly enabled a suitable method for separating the two otherwise indistinguishable forms of disaccharides. This is specifically surprising, since the choice of nucleophiles turned out to be essential for the present invention (other nucleophiles tested did not distinguish between the two forms). In contrast to other ways which could work in principle (e.g. acetolysis (e.g. sodium acetate in acetic acid)), only the method according to the present invention turned out to be applicable for enabling this separation. On the other hand, the present method also has its limits with respect to the linking geometry of the disaccharides. Whereas the method according to the present invention is very specific and practical for 1→4 disaccharides, it turned out to be non-satisfactory for 1→6 disaccharide (again, a surprising element of the present invention).

The present invention therefore teaches methods that can be applied
- to restrict to a minimum the formation of side products
- to separate D-gluco from D-manno azidonitrates and prepare them in pure form
- to prepare D-gluco and D-manno azidoacetates as suitable substrates for further processing; from these, not only azido sugars and amino sugars, but also special glycosyl donors such as halides and trichloroacetimidates may be prepared (Lemieux and Ratcliffe, *loc. cit*.).

Preferably, the method according to the present invention is applied to a mixture containing 1,2-diequatorial azidonitrate derived from cellobial acetate, wherein the mixture is treated with the nucleophile to obtain pure 1-axial-2-equatorial azidonitrate.

Preferably, the method according to the present invention is applied to a mixture containing 1,2-diequatorial azidonitrate derived from maltal acetate, wherein the mixture is treated with the nucleophile to obtain pure 1-axial-2-equatorial azidonitrate.

Preferably, the method according to the present invention is applied to a mixture containing 1,2-diequatorial azidonitrate derived from lactal acetate, wherein the mixture is treated with the nucleophile to obtain pure 1-axial-2-equatorial azidonitrate.

Preferably, the method according to the present invention is applied to a mixture containing α-azidonitrates and wherein pure β-azidoacetates are obtained by the action of the nucleophile, especially by the action of tetraalkylammonium nitrate.

According to a preferred embodiment, the nucleophile is selected from the group of tetrabutylammonium nitrate, tetraethylammonium nitrate, tetrapropylammonium nitrate, and tetrapentlylammonium nitrate, especially tetrabutylammonium nitrate. In fact, according to the present invention, "alkyl" may be methyl- to hexyl-, preferably ethyl- to pentyl-, especially pentyl- or butyl-.

The disaccharide consists preferably of pentoses, hexoses, heptoses, sialic acids, or mixtures thereof.

According to a specifically preferred embodiment, the disaccharide consists of two hexoses, especially of glucose, mannose, galactose, or mixtures thereof.

According to an advantageous embodiment of the present invention, obtaining the 1-axial-2-equatorial derivative from the treated mixture is performed by chromatography. This allows the application of established separation technologies in the present field which allows simple provision of the purified forms of the specific forms of the disaccharides. A specifically preferred chromatography is flash chromatography (i.e. chromatography with faster flow rates achieved by using a pump or by using compressed gas (e.g. air, nitrogen, or argon) to push the solvent through the column ("flash column chromatography")), as disclosed e.g. by Still et al., J. Org. Chem., 1978, 43 (14), pp 2923-2925; DOI: 10.1021/jo00408a041.

Accordingly, the present invention specifically relates to a method for separating chromatographically indistinguishable 1,2-diaxial from 1,2-diequatorial disaccharide azidonitrates, wherein the disaccharide is a 1→4 disaccharide, comprising the steps of
- providing a mixture of chromatographically indistinguishable 1,2-diaxial and 1,2-diequatorial disaccharide azidonitrates,
- treating the mixture with tetrabutylammonium nitrate thereby converting the 1,2-diequatorial disaccharide azidonitrate into a 1-axial-2-equatorial derivative which can be separated from the 1,2-diaxial disaccharide azidonitrate by chromatography, and
- obtaining the 1-axial-2-equatorial derivative and the 1,2-diaxial disaccharide azidonitrate from the treated mixture and optionally converting the 1-axial-2-equatorial derivative again to the 1,2-diequatorial disaccharide azidonitrate after obtaining the 1-axial-2-equatorial derivative.

According to another aspect, the present invention also relates to a pure preparation of a mannosazide or mannosamine derivative derived from cellobial acetate by azidonitration, wherein purity is defined as having at least 90% w/w, preferably at least 95% w/w, especially at least 98% w/w, content of the mannosazide or mannosamine derivative of all oligo-saccharides, especially of all di-saccharides.

According to another aspect, the present invention also relates to a pure preparation of a mannosazide or mannosamine derivatives derived from maltal acetate by azidonitration, wherein purity is defined as having at least 90% w/w, preferably at least 95% w/w, especially at least 98% w/w, content of the mannosazide or mannosamine derivative of all oligo-saccharides, especially of all di-saccharides.

According to another aspect, the present invention also relates to a pure preparation of a mannosazide or mannosamine derivatives derived from lactal acetate by azidonitration, wherein purity is defined as having at least 90% w/w, preferably at least 95% w/w, especially at least 98% w/w, content of the mannosazide or mannosamine derivative of all oligo-saccharides, especially of all di-saccharides.

The total of oligo-saccharides in the preparations according to the present invention may include oligo-saccharides with up to 30 saccharide units; usually, oligo-saccharides have 2 to 10 saccharide units.

According to another aspect, the present invention also relates to a pure 1,2-diaxial azidonitrate preparation obtainable by the method according to the present invention.

In the following, the preferred essential aspects of the invention are described for the azidonitration products of cellobial acetate. Analogous observations have been made for azidonitration of the disaccharide glycal acetates, maltal acetate and lactal acetate.

The products initially formed in the azidonitration of 1,4-linked disaccharide derivatives containing D-glucal as the reducing unit, namely the 1,2-diequatorial D-gluco **2** and the 1,2-diaxial D-manno-azidonitrates **3,** occur in ratios of 1:1 to 3:1. These products are chromatographically inseparable. By thin-layer chromatography (tlc) they are identified as one spot. According to the invention, they are isolated together as Fraction I by column chromatography on silica gel (Schemes 1 and 2; Shing and Perlin, *loc. cit*.).

According to the invention, care is being taken to avoid the degradation, under the reaction conditions, of the unstable 1,2-diequatorial product to form the β-D-gluco-(N-acetyl)glycosylamine **5** (Fraction III) and the α-D-gluco-(N-acetyl) glycosylamine **6** (Fraction IV; Shing and Perlin, *loc. cit*.). Presumably, these products are formed from the relatively unstable β-D-gluco azidonitrate **2** by nucleophilic substitution with the nitrate and ammonia formed by the reductive decomposition of the cerium ammonium nitrate complex (Scheme 1). N-acetylation is expected to occur spontaneously by the reaction of glycosylamines with any of the numerous O-acetyl groups in the system. To optimize the yield of Fraction I and minimize the loss of azidonitrate due to side reactions, the azidonitration reaction is performed as originally described by Lemieux and Ratcliffe (*loc. cit*.) with the following changes. 1. The reaction is stopped as soon as the starting glycal is consumed (Scheme 2). 2. Consumption of the glycal is assessed with the aid of tlc (silica gel 60 F₂₅₄, hexane:ethyl acetate, 2:3) and the anisaldehyde - sulfuric acid spray reagent. Upon consumption of the glycal, the characteristic dark purple coloration of the glycal (R_{F} 0.47) vanishes and is replaced by the light yellow/orange spots of Fraction I (R_{F} 0.63). In most cases, small amounts of the α-D-gluco azidonitrate (Fraction II) have formed by the time the glycal is consumed. Anisaldehyde staining of **4** is yellow and permits its distinction from **1.**

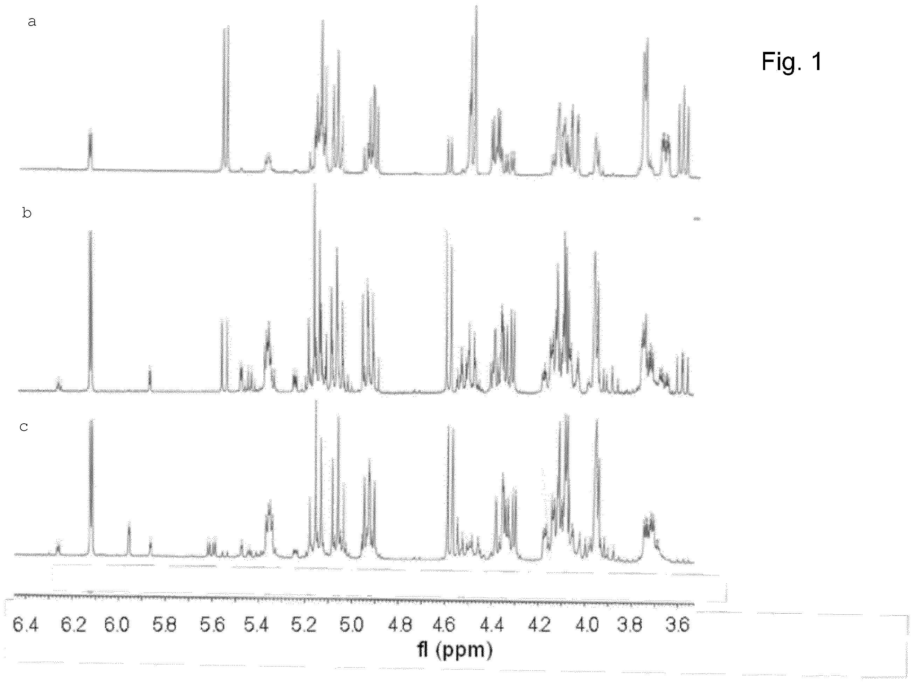

In a further aspect of the invention, the Fractions I and II are isolated separately by column chromatography on silica gel. Figure 1a shows a ¹H-NMR spectrum (400 MHz) of Fraction I as obtained by Shing and Perlin (*loc.cit.*). According to the invention, Fraction I is treated with excess tetrabutylammonium nitrate in anhydrous acetonitrile. During such treatment, ca. 90% of the unstable, 1,2-diequatorial β-D-gluco azidonitrate **2** are transformed into the stable, 1-axial-2-equatorial α-D-gluco azidonitrate **4** while the 1,2-diaxial α-D-manno- azidonitrate **3** remains substantially unchanged (Scheme 2). The larger part of the D- gluco product is now chromatographically isolated as the α-azidonitrate **4.** The remaining portion of Fraction I (Fraction IA) now contains the D-manno azidonitrate **3** in excess (gluco : manno, ca. 1:2; a partial ¹H-NMR spectrum [400 MHz] of Fraction IA obtained from the azidonitration of cellobial is shown in Figure 1b). Treatment of Fraction IA with tetrabutylammonium nitrate yields another small quantity of the α-D-gluco azidonitrate **4** plus Fraction IB which is practically pure α-D-manno azidonitrate **3** (Scheme 2; a ¹H-NMR spectrum (400 MHz) of the manno azidonitrate as obtained from cellobial is shown in Figure 1c). Conversion of **3** into intermediates suitable for further processing is best performed by conventional acetolysis (cf. below).

According to conventional protocols (Lemieux and Ratcliffe, *loc. cit*.), azidoacetates as suitable intermediates for further processing are obtained by acetolysis (sodium acetate in acetic acid). When this standard protocol is applied to the main product according to the invention, α-D-gluco azidonitrate **4,** a mixture of α-azidoacetate **7** (ca. 30%), β-azidoacetate **8** (ca. 30%) and unreacted α-azidonitrate **4** (ca. 40%) is obtained (Scheme 3).

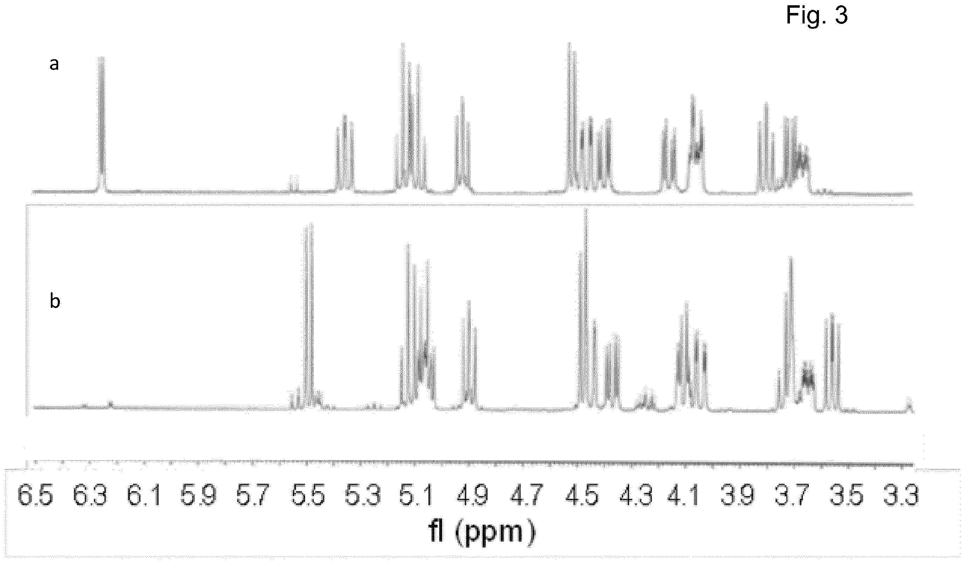

In a further aspect of the invention, the α-azidonitrate **4** is briefly treated with excess tetrabutylammonium acetate (40°C, dry acetonitrile, 15 min) to afford a near quantitative yield of the respective β-azidoacetate **8** (Scheme 4). A ¹H-NMR spectrum (400 MHz) of **8** obtained from cellobial is shown in Figure 3. From the β-azidoacetate **8 ,** free azido- (**9**), amino-(**10**) and acetamido sugars as well as glycosyl donors such as halides and trichloroacetimidates can be prepared by a multitude of methods known to those skilled in the art (Lemieux and Ratcliffe, *loc. cit.;* Scheme 5).

Treatment of the α-D-gluco azidonitrate **4** with tetrabutylammonium nitrate at equilibrium affords a ca. 10% yield of the pure β-D-gluco azidonitrate **2** (Scheme 5). Conventional acetolysis of the β-D-gluco azidonitrate **2** (sodium acetate in acetic acid) affords the α-D-gluco azidoacetate **7** in high yield.

The free disaccharide, β-D-galactopyranosyl-(1→4)-2-azido-2-deoxy-D-glucopyranose **9** is obtained from **8** by treatment with sodium methylate in methanol. In analogy to cellobiose, **9** can be subjected to enzyme-catalyzed conversions to form analogs of cellodextrins and cellulose. Catalytic hydrogenation of **9** affords amino derivatives of cellobiose, of interest for their potential activity against osteoarthritis.

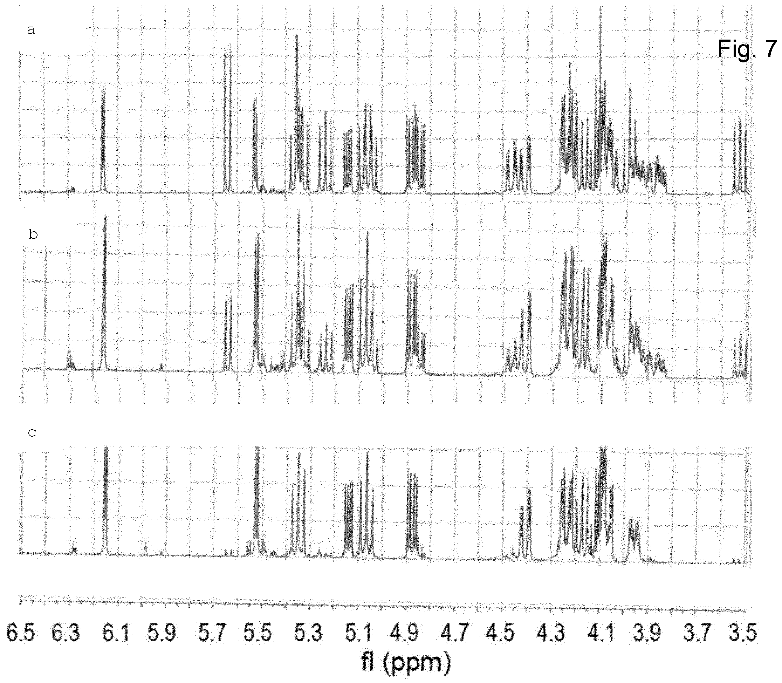

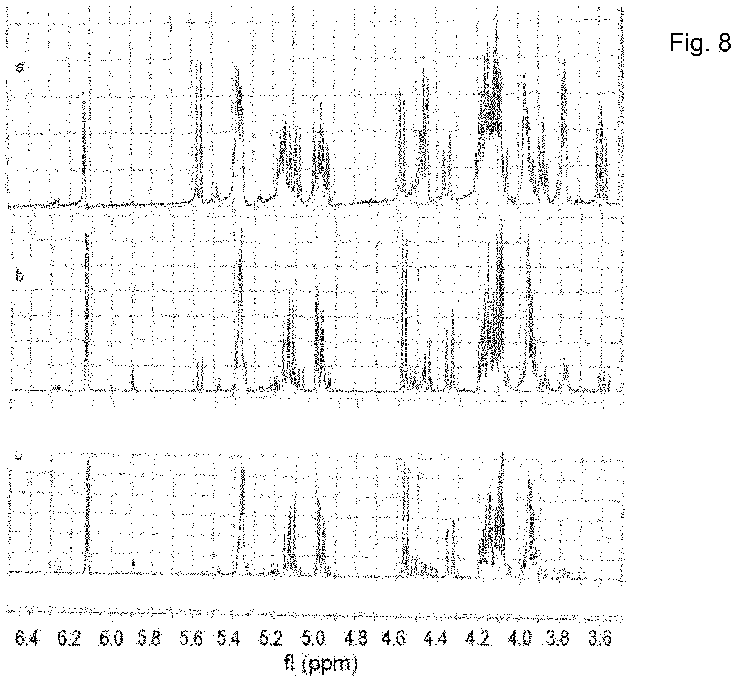

In a further aspect of the invention, it has been discovered that the methods that can be applied to separate D-gluco from D-manno acetates and prepare them in pure form, to restrict to a minimum the formation of side products, and to prepare D-gluco and D-manno azidoacetates as suitable substrates for further processing can be similarly applied to perform the corresponding conversions with the disaccharide glycal acetates, maltal acetate (Fig. 7) and lactal acetate (Fig. 8). Examples of products to be derived from maltosazide **11** are shown in Scheme 6. Compounds such as **16** and its N-acylated derivatives are of interest as nutrients, particularly in context with infant nutrition and artificial mother's milk. Free sugars such as **15** are analogs of maltose and are expected to be convertible, by enzyme-catalyzed processes, into nitrogen-containing maltodextrins, cyclodextrins and other derivatives of starch.

Products derived from lactosazide **17** are shown in Scheme 7. Compound **17** and its derivatives are nitrogen-containing analogues of lactose. They are starting materials for syntheses of artificial oligosaccharides used in replacements of mother's milk. Furthermore, free sugars such as **21** and **22** are candidates for modifiers or inhibitors of galectin-3 and other galectins. In addition, all compounds containing free glucosamine units are candidates for effecting epigenetic modification resulting in chondroprotective action.

Compound **17** and its N-acylated derivatives are nitrogen-containing analogues of lactose. They are starting materials for syntheses of oligosaccharides used in artificial replacements of mother's milk. Furthermore, free sugars such as **21** and **22** are candidates for modifiers or inhibitors of galectin-3 and other galectins.

During workup according to the invention of the azidonitration mixtures of cellobial acetate, maltal acetate and lactal acetate, minor amounts of mannosazide derivatives **23, 24** and **25** are obtained in practically pure form (Scheme 8). With the exception of **24,** these compounds are new and may be converted into azidoacetates **26, 27** and **28,** suitable for further processing. Potential targets are disaccharide derivatives of N-acetylmannosamine, potential inhibitors of NeuAc synthase and candidate anticancer agents (e.g. **29, 30** and **31**).

The present invention is further described by the following examples and the figures, yet without being limited thereto.
**Fig.1** shows the depletion of 2,3,4,6-tetra-O-acetyl-β-D-glucopyranosyl-(1→4)-3,6-di-O-acetyl-2-azido-2-deoxy-1-O-nitro-β-D-glucopyranose **2** from Fraction I by successive treatments with tetrabutylammonium nitrate (Scheme 2).
Fig.1a shows a partial ¹H NMR spectrum (400 MHz, CDCl₃) of Fraction I. The contents of gluco:manno isomer are estimated at ca. 3:1. Signals attributable to the β-gluco azidonitrate included δ 5.55 ppm (J ca. 8 Hz, H-1), 3×dd at ca. 5.2 - 4.9 ppm (H-3', 4' and 2' of the nonreducing glucose moiety), ca. 4.5 (d, overlapping with dd of H-6a; H-1'), 3.75 (broad m, H-4, H-5), ca. 3.65 (m, H-5') and 3.58 ppm (dd, H-2). These signals are seen to decrease in the part spectrum shown in Fig. 1b and to become negligibly small in the part spectrum shown in Fig. 1c.
Fig.1b shows a partial ¹H-NMR spectrum of Fraction IA, produced together with 2,3,4,6-tetra-O-acetyl-β-D-glucopyranosyl-(1→4)-3,6-di-O-acetyl-2-azido-2-deoxy-1-O-nitro-α-D-glucopyranose **4** by treatment of Fraction I with tetrabutylammonium nitrate (Scheme 2). In Fraction IA, the ratio of gluco:manno product is estimated as ca. 1:2. Depletion of the gluco azidonitrate **2** is noticeable by observing the significant decrease of the signals at δ 5.55, 4.9 (H-2'), 4.5 (H-1'), 3.75 (H-4, H-5), 3.65 and 3.58 ppm (H-5', H-2). Simultaneously, the signals due to the manno azidonitrate **3** have increased relative to Fig. 1a: δ 6.12 (d, *J* 3.2 Hz, H-1), 5.36 (m, H-3), 5.18 - 4.88 (3×dd, H-3', 4' and 2' of the nonreducing glucose moiety of **3**), 4.58 (d, *J* 8.0 Hz, H-1'), 4.09 (H-2), 3.95 (H-4, H-5) and 3.72 ppm (m, H-5').
   In Fig.1c, the signals due to **2** are practically absent and the partial NMR spectrum corresponds to that of the α-D-manno azidonitrate **3.**

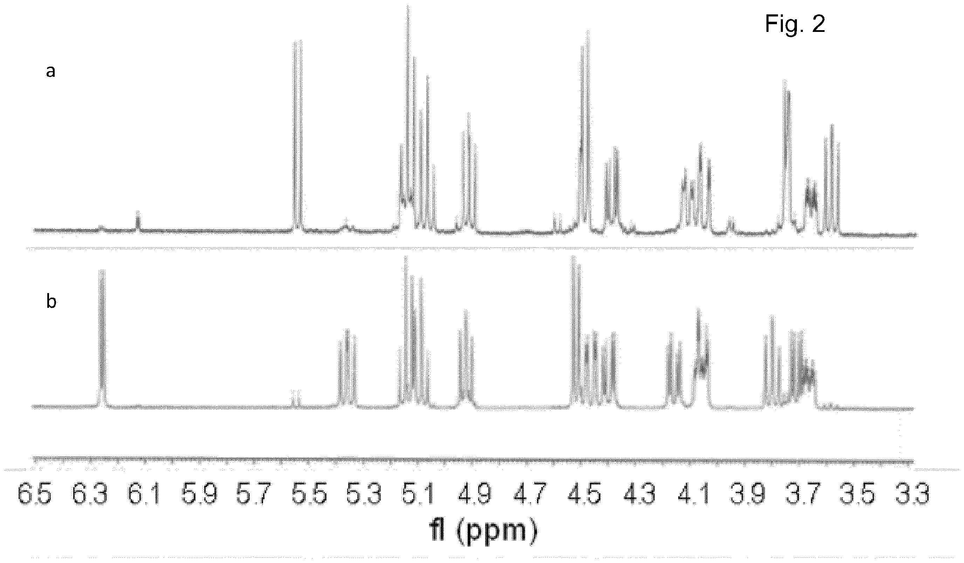

**Fig.2** shows the partial ¹H-NMR spectrum (400 MHz, CDCl₃) of the α-D-gluco azidonitrate **4** as prepared from the β-D-gluco azidonitrate **2** of Fraction I by treatment with tetrabutylammonium nitrate (Scheme 2). Upon conversion into the α-anomer **4,** the axial H-1 signal of **2** (doublet at δ ∼5.55 ppm, *J* ∼ 8Hz) experiences a downfield shift going to an equatorial position (δ 6.26 ppm, *J*∼ 4 Hz). Most significantly, the H-3 and H-5 of **4** are subject to an in-space electron withdrawing effect of the axial nitrate group, and their signals appear at δ 5.36 and 4.05 ppm (after 5.15 and 3.75 ppm in **2**). The signal due to H-4 in **4** is free of the overlapping H-5 resonance and shows the expected axial-axial couplings with H-3 and H-5. The adjacent signal of H-2 in **4** (dd, *j* ∼4, *J*∼9 Hz) shows the change from an axial-axial coupling in **2** to an axial - equatorial coupling in **4.** As expected, the signals attributed to the nonreducing glucopyranosyl moiety change very little upon transition from **2** to **4** (3×dd, 5.17 - 4.88 ppm, H-3', 4', 2'; d 4.53, H-1'; dd 4.4, H-6'a; dd 4.05, H-6'b and m 3.67 ppm, H-5').
**Fig.3** shows a partial ¹H-NMR spectrum (400 MHz, CDCl₃) of the β-D-gluco azidoacetate **8** (Scheme 4) prepared from the α-D-gluco azidonitrate **4** by treatment with tetrabutylammonium acetate. Treatment of the α-D-gluco azidonitrate derivative **4** (Fig. 3a) with tetrabutylammonium acetate (acetonitrile, 40°C, 20 min; Scheme 4) affords the β-O-acetyl derivative **8** in practically quantitative yield (Fig. 3b).

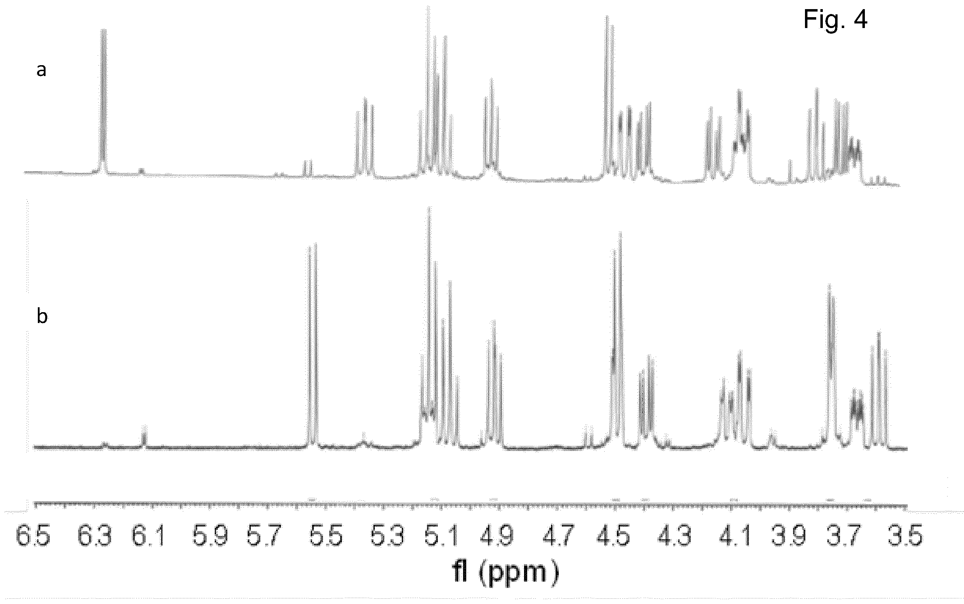

**Fig.4** shows a partial ¹H-NMR spectrum (400 MHz, CDCl₃) of the β-D-gluco azidonitrate **2** as obtained from the α-D-gluco azidonitrate **4** by treatment with tetrabutylammonium nitrate. Equilibration of the α-D-gluco azidonitrate **4** (Fig.4a) with an equal amount of tetrabutylammonium nitrate in dry acetonitrile (40°C, 2h) affords a 10% yield of the β-D-gluco azidonitrate **2** (Fig.4b) while 90% of the starting material is recovered unchanged.

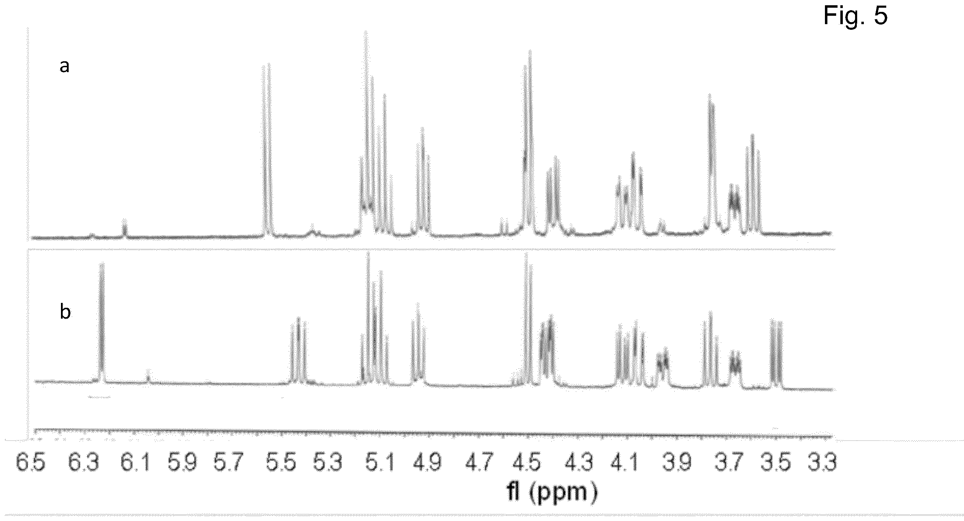

**Fig.5** shows a partial ¹H-NMR spectrum (400 MHz, CDCl₃) of the α-D-gluco azidoacetate **7** (Scheme 3, Scheme 5) obtained by treatment of the β-D-gluco azidonitrate **2** with sodium acetate in acetic acid. Treatment of the β-D-gluco azidonitrate **2** (Fig.5a) with sodium acetate in acetic acid affords the α-D-gluco azidoacetate **7** (Fig. 5b) in high yield. The presence of the anomeric acetate group is further confirmed by the appearance of seven lines for acetate carbonyl carbon atoms in the ¹³C-NMR spectrum of **7,** as compared to six lines in **2.**

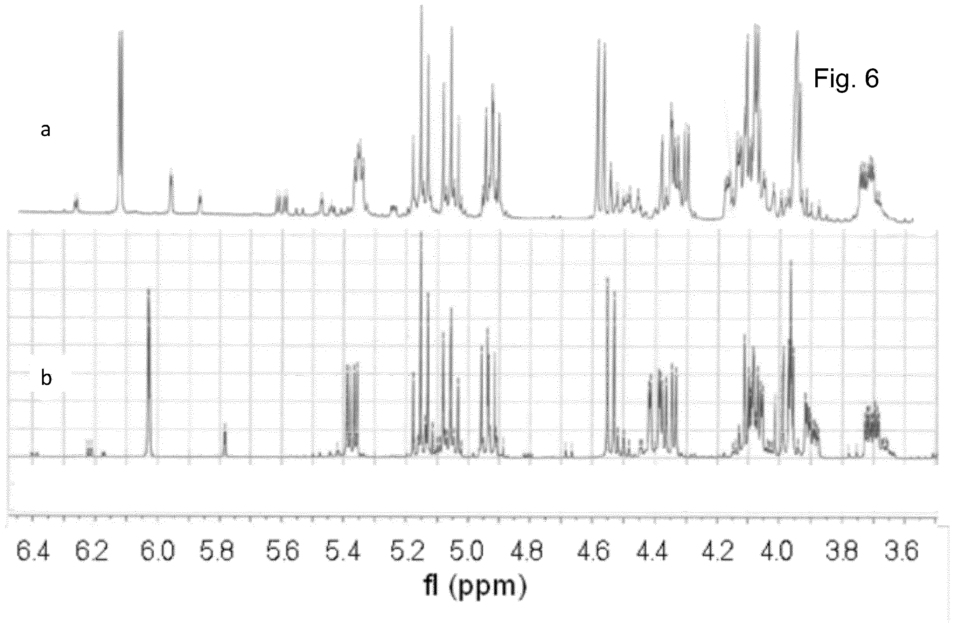

**Fig.6** shows a partial ¹H-NMR spectrum (400 MHz, CDCl₃) of the α-D-manno azidoacetate **26** (Scheme 8) obtained by treatment of the α-D-manno azidonitrate **3** with sodium acetate in acetic acid. Treatment of the α-D-manno-azidonitrate **3** (Fig.6a) with sodium acetate in acetic acid affords the α-D-manno azidoacetate **26** (Fig.6b) in high yield. The presence of the anomeric acetate group is further confirmed by the appearance of seven lines for acetate carbonyl carbon atoms in the ¹³C-NMR spectrum of **26,** as compared to six lines in **3.**
**Fig.7** shows depletion of the β-D-gluco azidonitrate from Fraction I obtained from maltal hexaacetate by successive treatments with tetrabutylammonium nitrate (Fig. 7a, Fraction I; Fig. 7b, Fraction IA; Fig. 7c, Fraction IB; for the corresponding scheme with cellobial hexaacetate, cf. Scheme 2).
**Fig.8** shows depletion of the β-D-gluco-azidonitrate from Fraction I obtained from lactal hexaacetate. The β-D-gluco azidonitrate is converted into the α-D-gluco azidonitrate by successive treatments with tetrabutylammonium nitrate (Fig. 8a, Fraction I; Fig. 8b, Fraction IA; Fig. 8c, Fraction IB; for the corresponding scheme with cellobial hexaacetate, cf. Scheme 2).

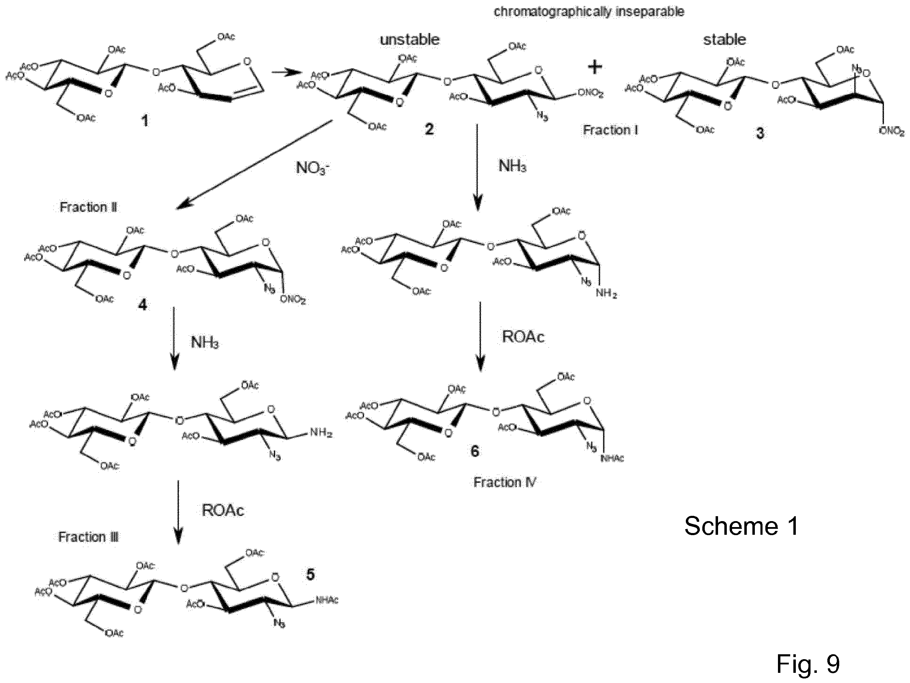

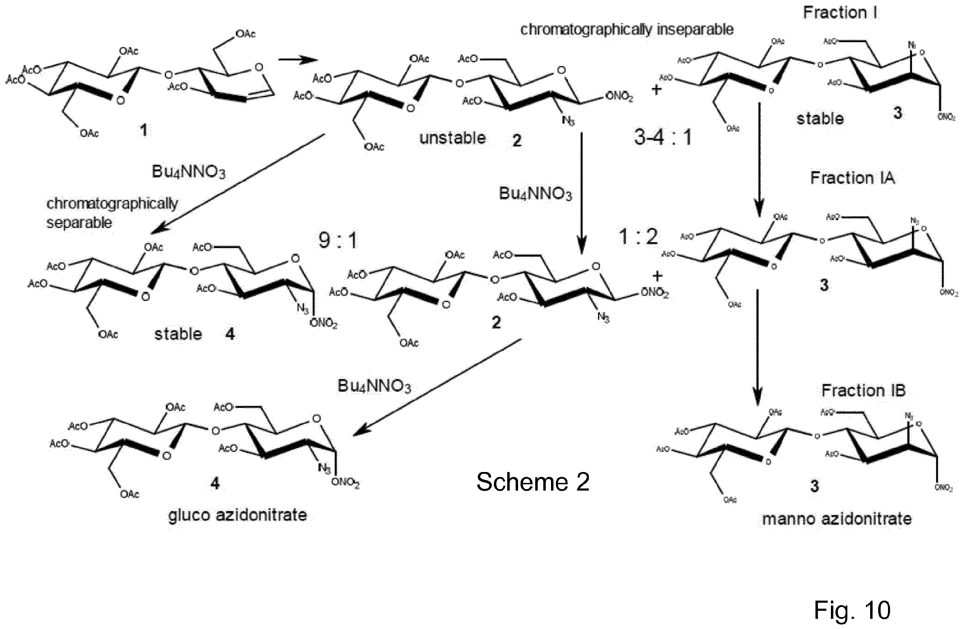

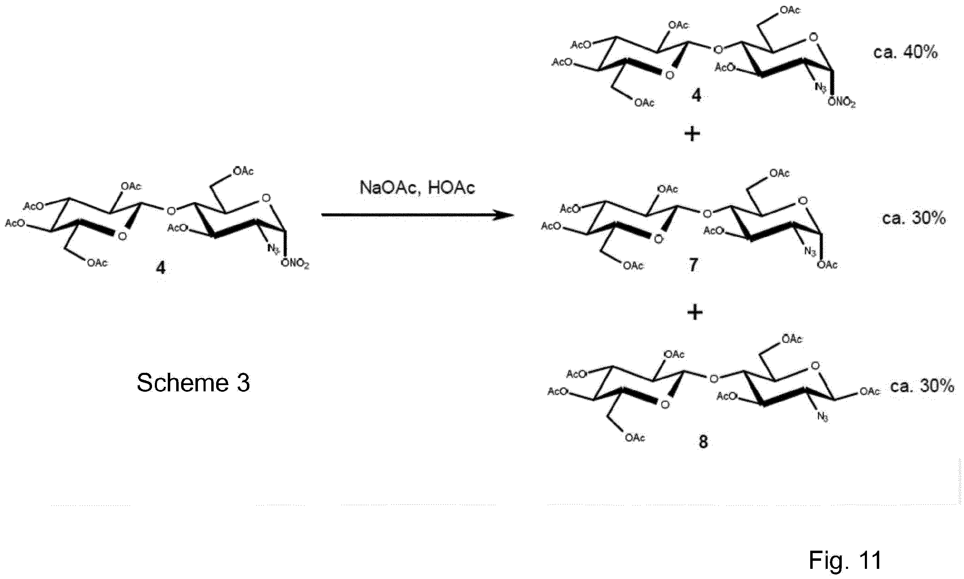

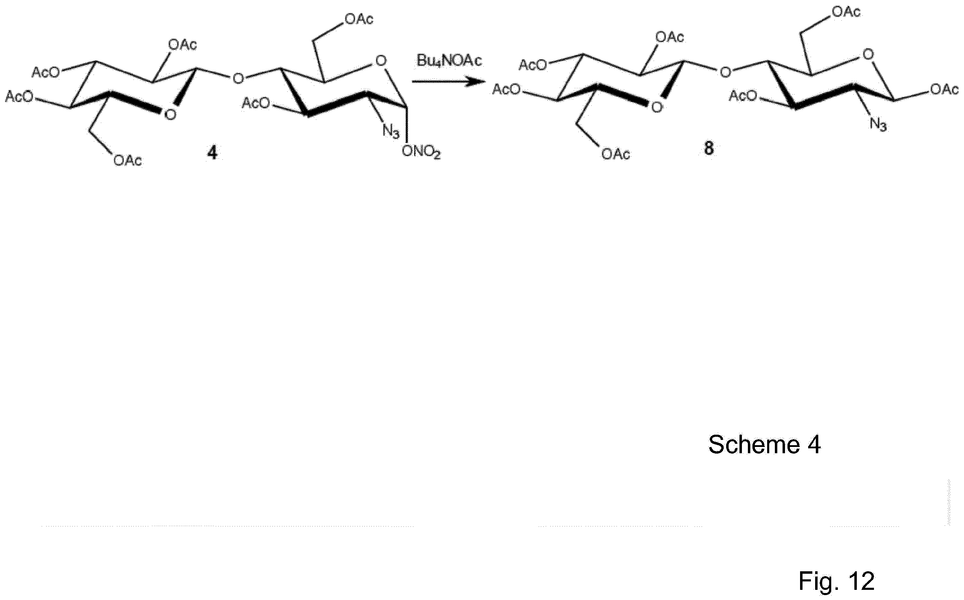

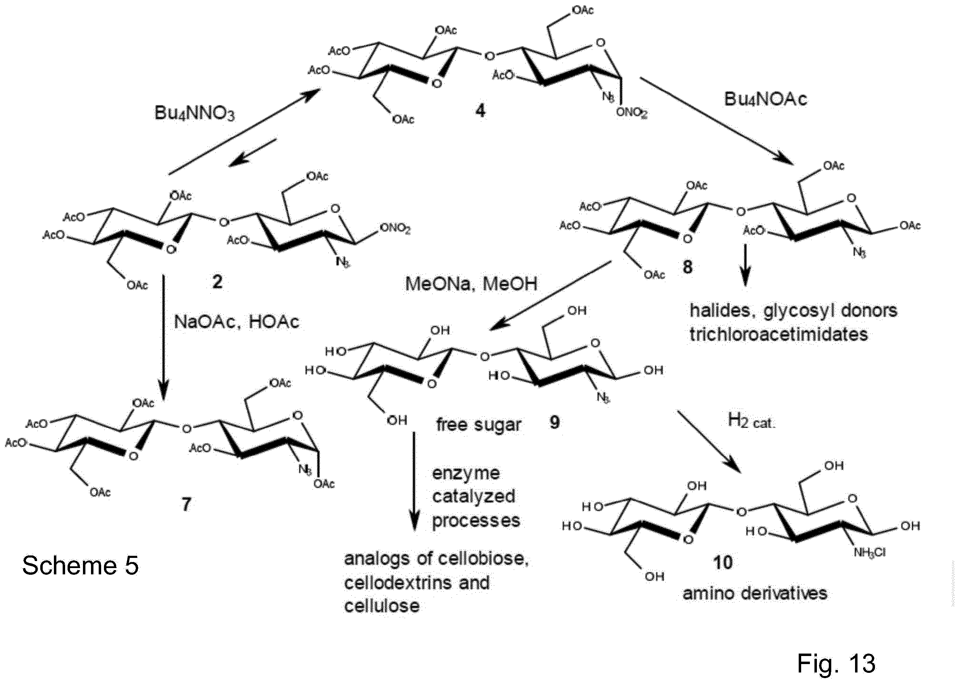

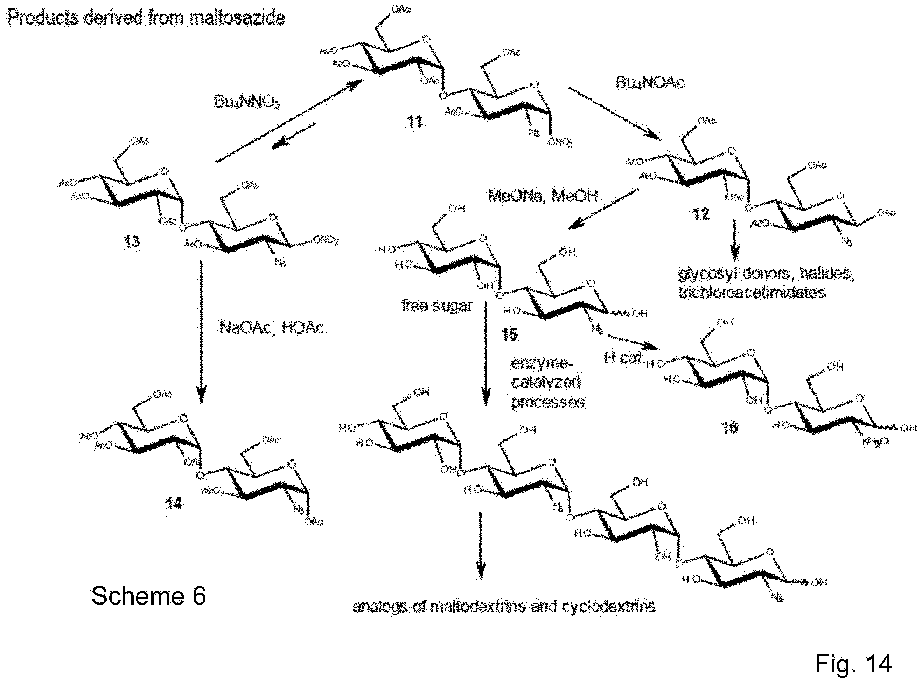

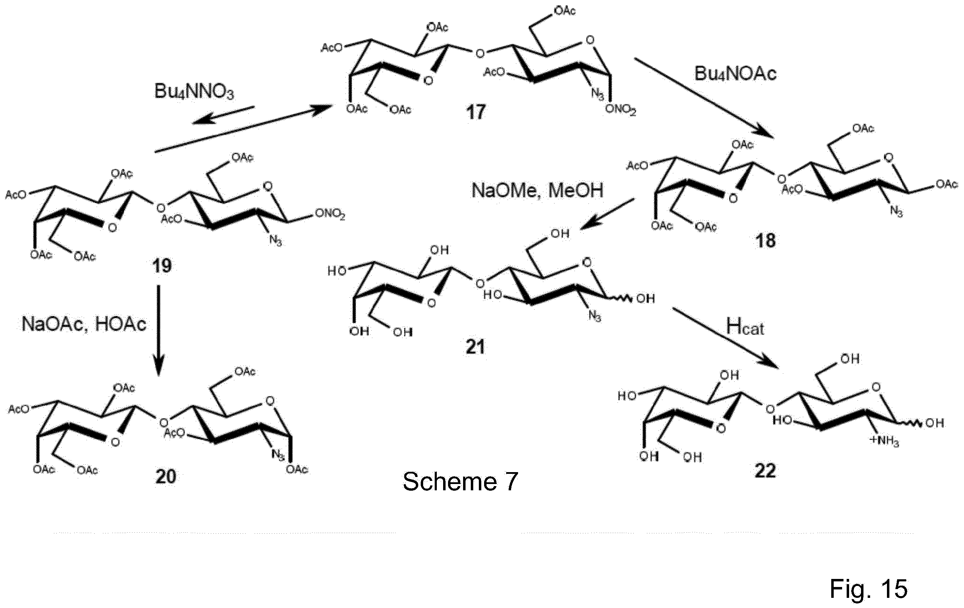

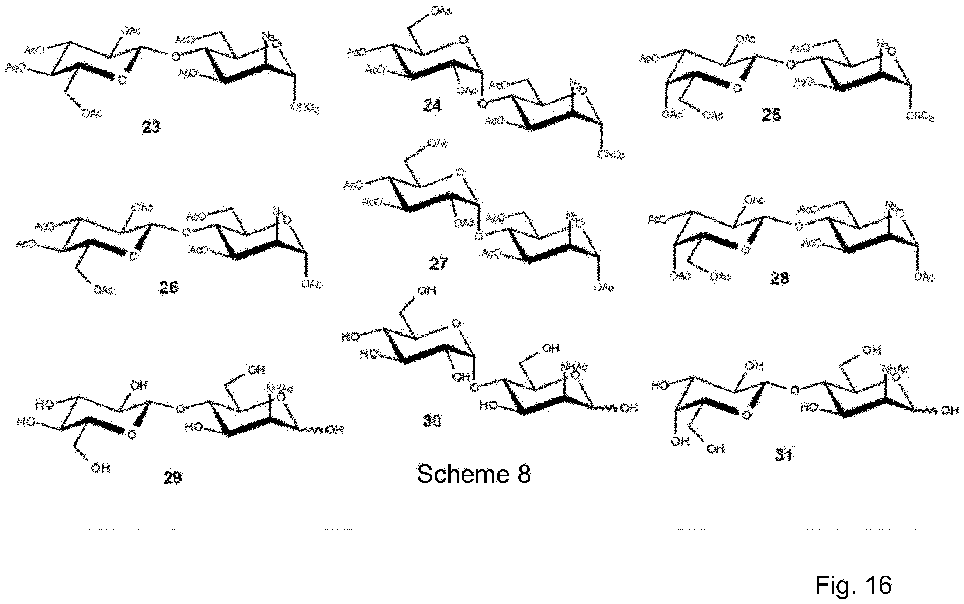

**Figs. 9 to 16** show Schemes 1 to 8.

### EXAMPLES:

### Example 1: Separation of the chromatographically indistinguishable β-D-gluco and α-D-manno azidonitrates 2 and 3 (Scheme 2). Stepwise conversion of the β-azidonitrate 2 into the α-anomer 4 and isolation of the α-D-manno azidonitrate 3 (Figures 1 and 2).

Compound **1** (2,3,4,6-tetra-O-acetyl-β-D-glucopyranosyl-[1→4]-3,6-di-O-acetyl-1,5-O-anhydro-1,2-eno-D-glucitol, 2 g, 3.57 mmoles) was dissolved in acetonitrile (20 mL) and the solution added to a dry mixture of ammonium cerium nitrate (4.88 g, 8.9 mmoles) and sodium azide (0.52 g, 8.0 mmoles) with stirring. The vessel was cooled to -15°C and the consumption of **1** followed by tlc (hexane:ethyl acetate, 2:3) using the anisaldehyde:sulfuric acid spray reagent. In ca. 3 - 4 h, the dark purple brown spot (R_{F} 0.47) due to **1** has disappeared in favor of an orange-brown spot (R_{F} 0.63) due to Fraction I. Care is taken to assure the complete consumption of **1** as the R_{F} value of **1** is similar to that of **4** and residual amounts of **1** will interfere later with the workup of the product mixture. However, with the anisaldehyde - sulfuric acid spray reagent, compound **4** shows distinct yellow to orange staining similar to Fraction I. The solvent acetonitrile is evaporated on a rotary evaporator and the product mixture distributed between diethyl ether (50 mL) and water (50 mL). The ether layer is dried over anhydrous magnesium sulfate and evaporated for a crude yield of 1.35 g (60%) of Fraction I plus a number of minor side products. The crude product is purified by column chromatography (30 × 420 mm, silica gel 60, hexane:ethyl acetate, 2:3).

Fraction I (330 mg, 0.50 mmoles) is dissolved in dry acetonitrile (5 mL) and tetrabutylammonium nitrate (350 mg, 1.15 mmoles) is added to the solution. The mixture is stirred at 40°C and the formation of **4** (R_{F} 0.59) from Fraction I (R_{F} 0.63) is followed by tlc (hexane:ethyl acetate, 2:3). After ca. 2 h, the solvent is evaporated and the residue distributed between chloroform and water (20 mL each). Compound **4** (110 mg, 33 %) is separated from Fraction IA (130 mg, 39 %) by column chromatography on silica gel (hexane:ethyl acetate, 2:3). Fig. 2 shows a comparison of partial ¹H-NMR spectra of **2** (Fig. 2a), the unstable main component of Fraction I, and the stable α-D-gluco azidonitrate derivative **4** (Fig. 2b).

Fraction IA (110 mg, 0.17 mmoles) is dissolved in dry acetonitrile (5 mL) and tetrabutylammonium nitrate (110 mg, 0.36 mmoles) is added to the solution. The mixture is stirred at 40°C and the formation of **4** (R_{F} 0.59) is followed by tlc (hexane:ethyl acetate, 2:3). After ca. 2 h, the solvent is evaporated and the residue distributed between chloroform and water (10 mL each). By column chromatography on silica gel, Fraction IB was isolated (50 mg, 45%), corresponding to the α-D-manno azidonitrate **3.** (R_{F} 0.63 in hexane:ethyl acetate, 2:3; Schemes 1 and 2). A partial ¹H-NMR spectrum of Fraction IB is seen in Figs. 1c and 6a. Further processing of Fraction IB to afford the crystalline α-D-manno azidoacetate **26** is described in Example 5 below.

### Example 2: Further processing I of the α-D-gluco azidonitrate 4; synthesis of the β-D-gluco azidoacetate 8 (Fig. 3).

To produce a homogeneous acetate intermediate for the further processing of **4,** the α-D-gluco azidonitrate is dissolved in anhydrous acetonitrile, and an equal amount of tetrabutylammonium acetate is added. The vessel is equipped with a drying tube, and the mixture is warmed to 40°C for 20 min, when tlc indicates quantitative conversion of **4.** The solvent is evaporated and the mixture distributed between chloroform and water. The chloroform phase contains a practically quantitative yield of the β-D-gluco azidoacetate **8.** Fig. 3 shows a comparison of the partial ¹H-NMR spectra of **4** (3a) and **8** (3b).

### Example 3: Preparation of the pure β-D-gluco azidonitrate 2 from the α-D-gluco azidonitrate 4 (Fig. 4).

In the presence of excess tetrabutylammonium nitrate, the α- and β-azidonitrates **4** and **2** will equilibrate in dry acetonitrile at 40°C to produce a mixture of 10% **2** and 90% **4.** This mixture is chromatographically separable and allows the production of pure, crystalline **2** (R_{F} 0.63 from **4** (R_{F} 0.59 in hexane:ethyl acetate, 2:3). Fig. 4 shows a comparison of partial ¹H-NMR-spectra between **4** (4a) and **2** (4b).

### Example 4: Further processing II of the β-D-gluco azidonitrate 2; synthesis of the α-D-gluco azidoacetate 7 (Fig. 5)

The β-D-gluco azidonitrate **2** (170 mg) is dissolved in glacial acetic acid (1.5 mL) and sodium acetate (100 mg) is added. The vessel is equipped with a drying tube and heated with magnetic stirring for 1 h at 100°C. The vessel is allowed to cool and a saturated solution (5 mL) of sodium bicarbonate is added. The aqueous phase is extracted with chloroform (3 × 10 mL), the chloroform phase dried over anhydrous magnesium sulfate and evaporated to afford the crystalline α-azidoacetate **7** in high yield. Fig. 5 shows partial ¹H-NMR spectra of **2** (5a) and **7** (5b). **Example 5:** Further processing of the α-D-manno azidonitrate **3** (Fraction IB); synthesis of the α-D-manno azidoacetate **26** (Fig. 6) .

The crude residue of **3** remaining after the depletion of **2** (as **4**) from Fraction I (50 mg, 0.075 mmoles) is dissolved in glacial acetic acid (3 mL) and sodium acetate (25 mg, 0.3 mmoles) is added. The vessel is equipped with a drying tube and the mixture heated to 100°C for 2h. Subsequently, the mixture is allowed to cool and is mixed with a saturated solution of sodium bicarbonate (10 mL). The mixture is extracted with chloroform (2×10 mL), the chloroform phase dried over magnesium sulfate and evaporated to give a crude yield of **26** (50 mg). Column chromatography (silica gel 60, 18×320 mm, hexane:ethyl acetate, 2:3) removes accompanying materials at ca. R_{F} 0.60 and affords pure **26** (R_{F} 0.51, colorless crystals, 30 mg [60%]; for a partial ¹H-NMR spectrum of **26,** see Fig. 6b).

## Claims

1. Method for separating chromatographically indistinguishable 1,2-diaxial from 1,2-diequatorial disaccharide azidonitrates, wherein the disaccharide is a 1→4 disaccharide, comprising the steps of
- providing a mixture of chromatographically indistinguishable 1,2-diaxial and 1,2-diequatorial azidonitrates,
- treating the mixture with a nucleophile, selected from the group of tetraalkylammonium salts, preferably tetraalkylammonium nitrate, and
- obtaining the 1-axial-2-equatorial derivative from the treated mixture.

2. Method according to claim 1, wherein the mixture contains 1,2-diequatorial azidonitrate derived from cellobial acetate and wherein the mixture is treated with the nucleophile to obtain pure 1-axial-2-equatorial azidonitrate.

3. Method according to claim 1, wherein the mixture contains 1,2-diequatorial azidonitrate derived from maltal acetate and wherein the mixture is treated with the nucleophile to obtain pure 1-axial-2-equatorial azidonitrate.

4. Method according to claim 1, wherein the mixture contains 1,2-diequatorial azidonitrate derived from lactal acetate and wherein the mixture is treated with the nucleophile to obtain pure 1-axial-2-equatorial azidonitrate.

5. Method according to any one of claims 1 to 4, wherein the mixture contains α-azidonitrates and wherein pure β-azidoacetates are obtained by the action of the nucleophile, especially by the action of tetraalkylammonium nitrate.

6. Method according to any one of claims 1 to 5, wherein the nucleophile is selected from the group of tetrabutylammonium nitrate, tetraethylammonium nitrate, tetrapropylammonium nitrate, and tetrapentlylammonium nitrate, especially tetrabutylammonium nitrate.

7. Method according to any one of claims 1 to 6, wherein the disaccharide consists of pentoses, hexoses, heptoses, sialic acids, or mixtures thereof.

8. Method according to any one of claims 1 to 7, wherein the disaccharide consists of two hexoses, especially glucose, mannose, galactose, or mixtures thereof.

9. Method according to any one of claims 1 to 8, wherein obtaining the 1-axial-2-equatorial derivative from the treated mixture is performed by chromatography, especially by flash column chromatography.

10. Method for separating chromatographically indistinguishable 1,2-diaxial from 1,2-diequatorial disaccharide azidonitrates, wherein the disaccharide is a 1→4 disaccharide, comprising the steps of
- providing a mixture of chromatographically indistinguishable 1,2-diaxial and 1,2-diequatorial disaccharide azidonitrates,
- treating the mixture with tetrabutylammonium nitrate thereby converting the 1,2-diequatorial disaccharide azidonitrate into a 1-axial-2-equatorial derivative which can be separated from the 1,2-diaxial disaccharide azidonitrate by chromatography, and
- obtaining the 1-axial-2-equatorial derivative and the 1,2-diaxial disaccharide azidonitrate from the treated mixture and optionally converting the 1-axial-2-equatorial derivative again to the 1,2-diequatorial disaccharide azidonitrate after obtaining the 1-axial-2-equatorial derivative.

11. Pure preparation of a mannosazide or mannosamine derivative derived from cellobial acetate by azidonitration, wherein purity is defined as having at least 90% w/w, preferably at least 95% w/w, especially at least 98% w/w, content of the mannosazide or mannosamine derivative of all oligo-saccharides, especially of all di-saccharides.

12. Pure preparation of a mannosazide or mannosamine derivatives derived from maltal acetate by azidonitration, wherein purity is defined as having at least 90% w/w, preferably at least 95% w/w, especially at least 98% w/w, content of the mannosazide or mannosamine derivative of all oligo-saccharides, especially of all di-saccharides.

13. Pure preparation of a mannosazide or mannosamine derivatives derived from lactal acetate by azidonitration, wherein purity is defined as having at least 90% w/w, preferably at least 95% w/w, especially at least 98% w/w, content of the mannosazide or mannosamine derivative of all oligo-saccharides, especially of all di-saccharides.

14. 1,2-diaxial azidonitrate preparation obtainable by a method according to any one of claims 1 to 10.
